(19) Europäisches Patentamt
European Patent Office
Office européen des brevets

(11) Publication number: 0 127 128
B1

(12) # EUROPEAN PATENT SPECIFICATION

(45) Date of publication of the patent specification. 25.02.87

(51) Int. Cl.[4]: C 07 C 93/06

(21) Application number: 84105894.4

(22) Date of filling: 23.05.84

(54) **Process for the conversion of the E isomer of 1,2-diphenyl-1-(4-(2-dimethylaminoethoxy)-phenyl)-1-butene to tamoxifen HCl.**

(30) Priority: 24.05.83 US 497551

(43) Date of publication of application: 05.12.84 Bulletin 84/49

(45) Publication of the grant of the patent: 25.02.87 Bulletin 87/9

(84) Designated Contracting States: AT BE CH DE FR GB IT LI LU NL SE

(56) References cited:
CH-A-624 923
DE-A-1 568 834

(73) Proprietor: Bristol- Myers Company, 345 Park Avenue, New York New York 10154 (US)

(72) Inventor: White, Dale A., 5 Treeline Avenue, Liverpool New York 13088 (US)

(74) Representative: Kinzebach, Werner, Dr., Patentanwälte Reitstötter, Kinzebach und Partner Sternwartstrasse 4 Postfach 86 06 49, D-8000 München 86 (DE)

EP 0 127 128 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convension).

LIBER, STOCKHOLM 1987

## Description

### Background Of The Invention

This invention relates to a process for the conversion of the E isomer of 1,2-diphenyl-1-[4-(2-dimethylaminoethoxy)phenyl]-1-butene to tamoxifen HCl.

Tamoxifen is a known non-steroidal anti-estrogen useful in the treatment of hormone-dependent tumors such as breast cancer. Tamoxifen is the Z or trans isomer of 1,2-diphenyl-1-[4-(2-dimethylaminoethoxy)-phenvl]-1-butene and has the formula:

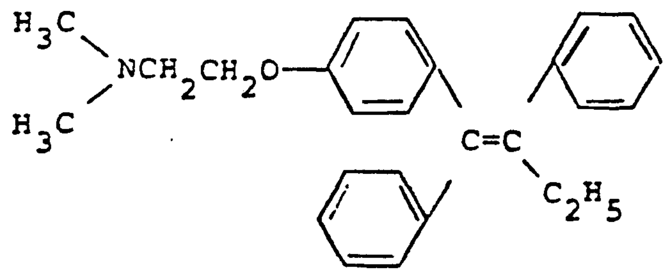

When produced by known processes the tamoxifen is formed in admixture with the corresponding E or cis isomer. Since the desired biological activity resides only in the Z isomer, the mixture of geometric isomers resulting from chemical synthesis must be separated.

A number of processes are known in the art for the production of tamoxifen and the corresponding E isomer. Thus, U.K. Patent No. 1,013,907 discloses a process which involves dehydration of an alkanol intermediate using an acid catalyst such as HCl to produce a mixture of tamoxifen and the corresponding E isomer. This process is illustrated by the following equation:

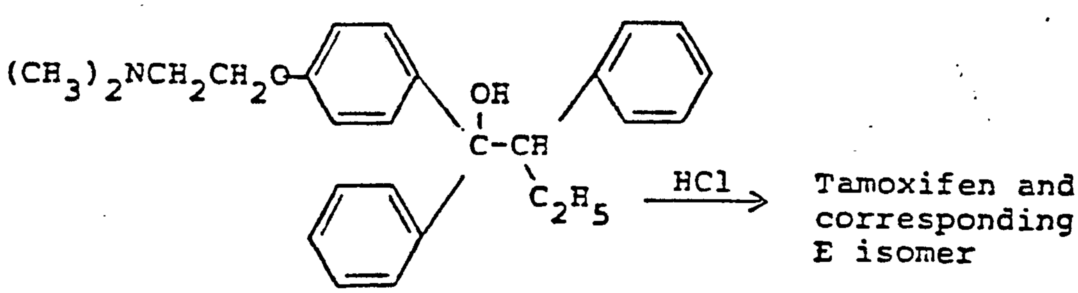


U.K. Patent No. 1,013,907 also discloses a process for the production of tamoxifen and the corresponding E isomer which involves alkylation of a phenolic intermediate. This process is illustrated by the following equation:

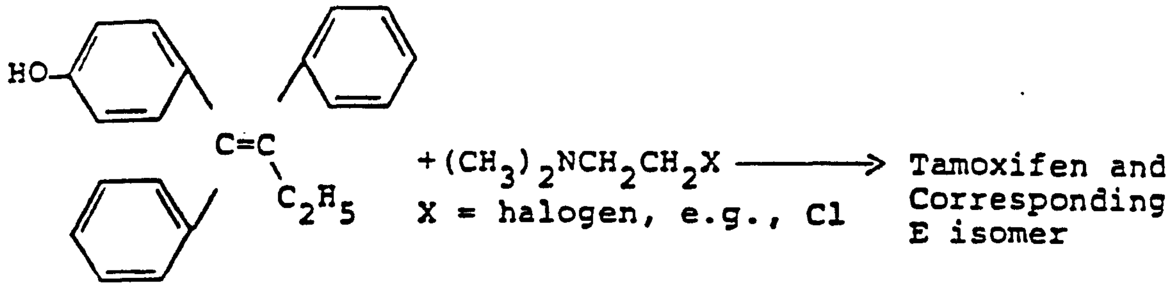


U.K. patent No. 1,354,939 discloses a process wherein a cyanide intermediate is dehydrogenated by an alkali metal amide to produce a mixture of tamoxifen and the corresponding E isomer. This process is illustrated by the following equation:

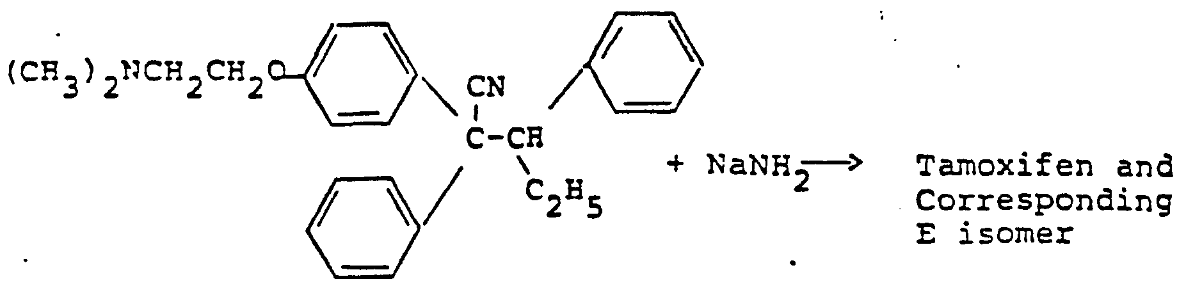


An abstract of Japanese application J5 2122-351, Derwent No. 83880Y/47, discloses the following reaction:

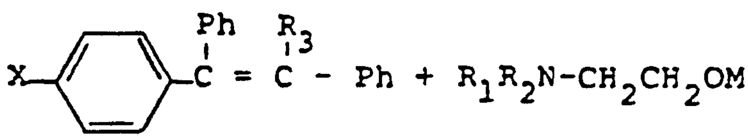

(II) (and/or isomer) (III)

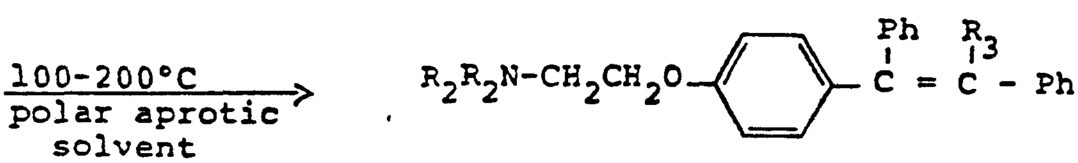


(I) (or isomer)

wherein $R_1$ and $R_2$ are the same or different 1-4 carbon alkyl groups, or $NR_1R_2$ is a heterocyclic group; $R_3$ is a 1-4 carbon alkyl group; X is halogen; and M is H or alkali metal, preferably Na or K.

A number of different procedures for separating tamoxifen from the corresponding E isomer are also known in the art. Canadian Patent No. 1,064,629, Canadian Patent No. 1,072,093, Chemical Abstracts, Vol. 67, Abstract 90515g (1967) and Chemical Abstracts, Vol. 71, Abstract 3134y (1969) all disclose separating tamoxifen from the corresponding E isomer by fractional crystallization. Canadian Patent No. 1,064,629 also discloses the chromatographic separation of these mixed isomers as do Burns et al in Journal of Labelled Compounds and

Radiopharmaceuticals, Vol. XIX, pages 229-239 and 503-523 (1981). In the prior art isomer separation processes, the E isomer of 1,2-diphenyl-1-[4-(2-dimethylaminoeth-oxy)phenyl]-1-butene is wasted since it does not possess the desired biological activity. Thus, the amount of tamoxifen which can be recovered from a given process for the production of a mixture of E and Z isomers of 1,2-diphenyl-1-[4-(2-dimethylaminoethoxy)phenyl]-1-butene-is severely limited.

**Summary Of The Invention**

It has now been discovered that the E isomer of 1,2-diphenyl-1-[4-(2-dimethylaminoethoxy)phenyl]-1-butene can be converted to tamoxifen hydrochloride by a process which comprises heating the E isomer of 1,2-diphenyl-1-[4-(2-dimethylaminoethoxy)phenyl]-1-butene, either alone or in admixture with tamoxifen, in hydrochloric acid at a temperature within the range of 35-60°C until substantial conversion of the E isomer of 1,2-diphenyl-1-[4-(2-di-methylaminoethoxy)phenyl]-1-butene to tamoxifen is accomplished. In this temperature range, i.e., 35-60°C, the tamoxifen precipitates from the reaction mixture as the HCl salt while most of the HCl salt of the E isomer of 1,2-diphenyl-1-[4-(2-dimethylaminoethoxy)phenyl]-1-butene stays in solution. The length of time for this conversion can be determined by HPLC or NMR. The tamoxifen HCl is recovered from the resultant slurry. It can be further purified by conventional procedures, e.g., by chromatography or fractional crystallization.

**Detailed Description Of The Invention**

The E isomer of 1,2-diphenyl-1-[4-(2-dimethyl-aminoethoxy) phenyl]-1-butene which is to be converted to tamoxifen hydrochloride can be obtained from any source, e.g., any of the previously discussed procedures for the preparation and separation of tamoxifen. Tamoxifen can also be present in any proportion since it will precipitate out of the hydrochloric acid solution along with the tamoxifen that results from the conversion of the E isomer of 1,2-diphenyl-1-[4-(2-dimethylaminoethoxy)phenyl]-1-butene. The E isomer of 1,2-diphenyl-1-[4-(2-di-(2-dimethylaminoethoxy)phenyl]-1-butene can be added to the hydrochloric acid in the form of the free base, in which case it will be converted to the HCl salt along with the tamoxifen during the conversion process; or it can be added to the hydrochloric acid in the form of the HCl salt. If the E isomer of 1,2-diphenyl-1-[4-(2-dimethylaminoethoxy)phenyl]-1-butene is in the form of a salt other than the HCl salt, e.g., acetate or citrate, it is preferred to convert it to the free base before adding it to the hydrochloric acid. Thus, the presence of acetate or citrate salts can cause some solubility changes that inhibit the isomer conversion. The free base can be formed from acid salts in a methylene chloride-aqueous system quite readily. The phases only need then to be separated and the methylene chloride stripped from the rich organic layer to leave the base as input to the isomerization reaction. Without conversion to the base, acid salts may require some modification in the temperature range to achieve crystallization of the tamoxifen.

The hydrochloric acid used in the practice of this invention should be at least 6 N, preferably at least 9 N, and most preferably is 12 N. The conversion can be conducted as either a batch or a continuous process. In a batch process, the E isomer of 1,2-di-phenyl-1-[4-(2-dimeethylaminoethoxy)phenyl]-1-butene is heated in the hydrochloric acid until substantial conversion to tamoxifen is completed, heating is then discontinued and the tamoxifen is separated from the resultant slurry. In a continuous process, a portion of the slurry is intermittently removed, tamoxifen is recovered as by filtration, and the hydrochloric acid from which tamoxifen has been separated is recycled to the conversion zone. It is preferred to heat the E isomer of 1,2-di-phenyl-1-[4-(2-dimethylaminoethoxy)phenyl]-1-butene in hydrochloric acid in the conversion zone for at least eight hours.

The temperature within the range of 35-60°C should be selected to insure crystallization of tamoxifen hydrochloride from solution while maintaining the E isomer of 1,2-diphenyl-1-[4-(2-dimethylaminoethoxy)-phenyl]-1-butene in solution. For relatively impure solutions, lower temperatures can be used. For a relatively pure mixture of tamoxifen and the E-isomer of 1,2-diphenyl-1-[4-(2-dimethylaminoethoxy)phenyl]-1-butene, a temperature within the range of 50-55°C is preferred.

The tamoxifen HCl can be recovered from the slurry by any conventional procedure, e.g., by filtration or centrifugation. It can then be purified to remove any E isomer of 1,2-diphenyl-1-[4-(2-dimethylaminoethoxy)-phenyl]-1-butene which precipitated from the hydrochloric acid solution. This is conveniently accomplished by selective crystallization from a suitable solvent such as MIBK (methylisobutylketone). The tamoxifen HCl can be converted to the free base or to another salt, e.g., the citrate which is the commercial form, by known techniques. The practice of this invention will result in a substantial cost reduction in the preparation of tamoxifen.

The following examples illustrate the best modes contemplated for carrying out the process of this invention:

**Example 1**

A mixture of tamoxifen hydrochloride and the hydrochloride salt of the E isomer of 1,2-diphenyl-1-[4-(2-dimethylaminoethoxy)phenyl]-1-butene (2.0 g, 4.9 mmole) with an E to Z isomer ratio of approximately 80:20 was added to 12 N hydrochloric acid (20 ml, 0.24 moles). A partial solution occurred which was stirred for one hour at which time a slurry began to form. The slurry was warmed to 55°C and held at that temperature for 19 hours. At this time, the isomer ratio was checked by HPLC (high pressure liquid chromatography). The ratio of Z to E isomer was found to be 94:6. The slurry was vacuum stripped to a pasty slurry and diluted with 60 ml of MIBK. Once again the slurry was stripped under vacuum and 60 ml of MIBK were added. The resulting slurry was warmed to form a solution (about 80°C) and then allowed to cool to re-precipitate the product. This slurry was sstirred for two hours, filtered, washed with MIBK, then heptane, and dried in an air oven at 45°C. There was thus obtained 1.54 g (3.77 mmole) of product which, by HPLC, was determined to be 100% tamoxifen HCl.

**Example 2**

An oil obtained as a by-product in a process for the separation of tamoxifen HCl from the HCl salt of the E isomer of 1,2-diphenyl-1-[4-(2-dimethylamino -ethoxy)phenyl]-1-butene and containing 8.297 g of the mixture of isomers in a E to Z ratio of 72:28 was mixed with 57 ml of 12 N hydrochloric acid. The batch was stirred and warmed to 55°C. After stirring at this temperature overnight, the Z to E isomer ratio was found to be greater than about 90:10. The batch was vacuum stripped to a pasty solid using a bath temperature of about 55-60°C. MIBK (200 ml) was added and vacuum stripping was continued until water was no longer present in the distillate. Additional MIBK (100 ml) was added during the stripping operation. The volume was adjusted to 250 ml by the addition of MIBK and the slurry was warmed to 80°C to form a solution. The solution was then cooled to 10°C over one and one-half hours and allowed to crystallize for four hours. The slurry was filtered and washed with 20 ml of cold MIBK and 20 ml of heptane. The cake was dried in an air oven at 45°C. The dry weight of the product was 7.7 g which was 100% tamoxifen HCl. The yield of tamoxifen HCl was 92.8% based upon the amount of isomer mixture present in the starting oil.

**Example 3**

In order to determine the effect of varying the normality of hydrochloric acid solution, a series of runs were conducted. In each run, 2.0 g of a mixture of tamoxifen hydrochloride and the hydrochloride salt of the E isomer of 1,2-diphenyl-1-[4-(2-dimethylaminoethoxy)-phenyl]-1-butene were added to 20 ml of hydrochloric acid solutions of varying strengths. The starting material contained 3.65% by weight of Z isomer and 96.35% by weight of E isomer. Each solution was agitated at 55°C for 18 hours and then assayed by HPLC to determine the change in the ratio of isomers. The results are set forth below:

| Acid Strength | Percent By Weight Z Isomer | Percent By Weight E Isomer |
|---|---|---|
| 1.0 N HCl | 6.7 | 93.3 |
| 3.0 N HCl | 7.0 | 93.0 |
| 6.0 N HCl | 8.9 | 90.1 |
| 9.0 N HCl | 62.0 | 38.0 |
| 12.0 N HCl | 95.8 | 4.2 |

## Claims

1. A process for the production of tamoxifen hydrochloride which comprises heating the E isomer of 1,2-diphenyl-1-[4-(2-dimethylaminoethoxy)phenyl]-1-butene in hydrochloric acid at a temperature within the range of 35-60°C until substantial conversion of the E isomer of 1,2-diphenyl-1-[4-(2-dimethylaminoethoxy)phenyl]-1-butene to tamoxifen hydrochloride is accomplished and tamoxifen hydrochloride crystallizes and recovering the resultant crystallized tamoxifen hydrochloride.

2. A process as defined in claim 1 wherein said E isomer of 1,2-diphenyl-1-[4-(2-dimethylaminoethoxy)phenyl]-1-butene is added to said hydrochloric acid in admixture with tamoxifen.

3. A process as defined in claim 2 wherein said E isomer of 1,2-diphenyl-1-[4-(2-dimethylaminoethoxy)phenyl]-1-butene and tamoxifen are added to the hydrochloric acid in the form of the hydrochloride salt.

4. A process as defined in anyone of claims 1, 2 or 3 wherein said heating is conducted at a temperature within the range of 50 - 55°C.

5. A process as defined in anyone of claims 1, 2 or 3 wherein said heating in hydrochloric acid is conducted for at least eight hours.

6. A process as defined in anyone of claims 1, 2 or 3 wherein said hydrochloric acid is at least 6 N.

7. A process as defined in anyone of claims 1, 2 or 3 wherein said hydrochloric acid is at least 9 N.

8. A process as defined in anyone of claims 1, 2 or 3 wherein said hydrochloric acid is 12 N.

## Patentansprüche

1. Verfahren zur Herstellung von Tamoxifen-Hydrochlorid, wobei man das E-Isomer von 1,2-Diphenyl-1-[4-(2-dimethyl -aminoethoxy)phenyl]-1-buten in Salzsäure bei einer Temperatur im Bereich von 35 bis 60°C erhitzt, bis eine wesentliche Umwandlung des E-Isomers von 1,2-Diphenyl-1-[4-(2-dimethylaminoethoxy)phenyl]-1-buten in Tamoxifen-Hydrochlorid erfolgt ist und Tamoxifen-Hydrochlorid kristallisiert, und man das erhaltene kristallisierte Tamoxifen-Hydrochlorid gewinnt.

2. Verfahren nach Anspruch 1, wobei das E-Isomer von 1,2-Diphenyl-1-[4-(2-dimethylaminoethoxy)phenyl]-1-buten in Mischung mit Tamoxifen zur Salzsäure gegeben wird.

3. Verfahren nach Anspruch 2, wobei das E-Isomer von 1,2-Diphenyl-1-[4-(2-dimethylaminoethoxy)phenyl]-1-buten und Tamoxifen in Form des Hydrochlorids zu der Salzsäure gegeben werden.

4. Verfahren nach einem der Ansprüche 1, 2 oder 3, wobei das Erhitzen bei einer Temperatur im Bereich von 50 bis 55°C durchgeführt wird.

5. Verfahren nach einem der Ansprüche 1, 2 oder 3, wobei das Erhitzen in Salzsäure wenigstens acht Stunden durchgeführt wird.

6. Verfahren nach einem der Ansprüche 1, 2 oder 3, wobei die Salzsäure wenigstens 6 N ist.

7. Verfahren nach einem der Ansprüche 1, 2 oder 3, wobei die Salzsäure wenigstens 9 N ist.

8. Verfahren nach einem der Ansprüche 1, 2 oder 3, wobei die Salzsäure 12 N ist.

## Revendications

1.- Un procédé de préparation de chlorhydrate de tamoxifen qui comprend le chauffage de l'isomère E du 1,2-diphényl-1-[4-(2 diméthylaminoéthoxy) phényl]-1- butène dans l'acide chlorhydrique à une température comprise entre 35 et 60°C jusqu'à obtention d'une transformation importante de l'isomère E du 1,2-diphényl-1-[4-(2 diméthylaminoéthoxy)-phényl]-1- butène en chlorhydrate de tamoxifen et cristallisation de ce dernier, et la récupération du chlorhydrate cristallisé résultant du tamoxifen.

2.- Un procédé selon la revendication 1 dans lequel ledit isomère E du 1,2-diphényl-1-(4-(2 diméthylaminoéthoxy) phényl)-1- butène est ajouté audit acide chlorhydrique mélangé au tamoxifen.

3.- Un procédé selon la revendication 2 dans lequel ledit isomère E du 1,2-diphényl-1-[4-(2 diméthylaminoéthoxy) phényl]-1-butène et le tamoxifen sont ajoutés à l'acide chlorhydrique sous forme de chlorhydrate.

4.- Un procédé selon l'une quelconque des revendications 1 à 3, dans lequel ledit chauffage est effectué à une température comprise entre 50 et 55°C.

5.- Un procédé selon l'une quelconque des revendications 1 à 3 dans lequel ledit chauffage dans l'acide chlorhydrique est poursuivi pendant une durée de huit heures au moins.

6.- Un procédé selon l'une quelconque des revendications 1 à 3 dans lequel ledit acide chlorhydrique est de normalité au moins égale à 6 N.

7.- Un procédé selon l'une quelconque des revendications 1 à 3 dans lequel ledit acide chlorhydrique est de normalité au moins égale à 9 N.

8.- Un procédé selon l'une quelconque des revendications 1 à 3 dans lequel ledit acide chlorhydrique a une normalitè égale à 12 N.